# EUROPEAN PATENT SPECIFICATION

(11) **EP 0 527 804 B1**
(45) Date of publication and mention of the grant of the patent: **15.03.2000**
(21) Application number: 91908358.4
(22) Date of filing: 10.04.1991
(51) Int. Cl.: C12N 15/00, C12P 21/04, A61K 48/00, C12N 9/00, C12N 15/12

(54) **GENE THERAPY FOR CELL PROLIFERATIVE DISEASES**
GENTHERAPIE GEGEN KRANKHEITEN DIE DIE ZELLTEILUNG BETREFFEN
THERAPIE GENETIQUE CONTRE DES MALADIES PROLIFERATIVES CELLULAIRES

(30) Priority: 10.04.1990 US 507417
(43) Date of publication of application: 24.02.1993
(60) Divisional application: 99113411.5
(73) Proprietor: CANJI, Inc., San Diego, CA 92121 (US)
(72) Inventor: FUNG, Yuen, Kai, Los Angeles, CA 90039 (US); MURPHREE, Alan, Linn, Los Angeles, CA 90039 (US)
(74) Representative: Wilkinson, Stephen John
(86) International application number: US9102478
(87) International publication number: WO9115580

(56) References cited:
- EP-A- 0 375 408
- EP-A- 0 475 623
- WO-A-90/05445
- WO-A-91/13989
- WO-A-91/18625
- US-A- 4 740 463
- JOURNAL OF CELLULAR BIOCHEMISTRY vol. SUPPL, no. 12A, 17 January 1988, NEW YORK USA page 217 T'ANG, A. ET AL. 'Inactivation of the human retinoblastoma gene in retinoblastoma and other tunors'
- JOURNAL OF CELLULAR BIOCHEMISTRY vol. SUPPL, no. 12A, 17 January 1988, NEW YORK USA page 217 T'ANG, A. ET AL. 'Structural feature and expression of the human retinoblastoma cDNA clone'
- Science, Volume 247, issued 09 February 1990, R. BOOKSTEIN et al., "Suppression of Tumorigenicity of human prostate carcinoma cells by replacing a mutates RB gene", pages 712-715, see entire article.
- Science, Volume 242, issued 16 December 1985, H.J.S. HUANG et al., "Suppression of the Neoplastic phenotype by replacement of the RB gene in human cancer cells", pages 1563-1566, see entire article.
- EMBO Journal, Volume 8, Number 12, issued 1989, M. COTTEN et al., "Ribozyme mediated destruction of RNA in vivo", pages 3861-3866, see entire article.
- Proceedings National Academy of Sciences, Volume 86, issued December 1989, F.H. CAMERON et al., "Specific gene suppressing by engineered ribozymes in monkey cells", pages 9139-9143, see entire article.
- Nature, Volume 334, issued 18 August 1988, J. HASELOFF et al., "Simple RNA enzymes with new and highly specific endoribonuclease activities", pages 585-591, see entire article.

## Description

### Field of the Invention

The present invention relates to a therapeutic modality for treatment of cell proliferative diseases comprising the insertion of a negatively-acting growth regulatory (NGR) element into a proliferating cell.

### Background of the Invention

Pathological conditions resulting in inappropriate local proliferation of cells are a common cause of human disease. Benign human diseases differ from malignant processes (cancer) primarily by the inability to spread from one part of the body to another and their generally slower growth rate. Both can kill, blind and maim. Unwanted internal adhesions and scarring after abdominal surgery can lead to bowel strangulation and death. Blindness from diabetes mellitus results from the inappropriate growth of new blood vessels inside the eye. Benign neurofibromas cause disfiguration. A skin disease like psoriasis is a life-long debilitating process resulting from the inappropriate overgrowth of otherwise normal cells. Scores of different human diseases can be assigned to this category.

Diseases resulting from inappropriate local proliferation of cells will be referred to in the collective as Benign Proliferative Diseases (BPD). When the cell predominating in the overgrowth population is the fibroblast, the diseases in the subgroup are known as "fibroproliferative" disorders. When the predominating cell is vascular endothelium (a cell type which lines the inner surface of blood vessels), the subgroup is known as "vasoproliferative" disorders. The inappropriate overgrowth of epithelium results in serious skin diseases. Currently, treatment of these benign proliferative diseases frequently requires tissue ablation with one or more of the following treatments: surgery, radiation, and/or chemotherapy. These treatments are all generally attended by damage to normal as well as pathological tissues. Other local treatment modalities, such as laser or local cryotherapy, are rarely successful.

### Regulation of Cell Proliferation

The control of cell proliferation is a complex process which has only just begun to be understood in recent years. Whether a cell is to grow or not depends on the balance of the expression of negatively-acting and positively-acting growth elements. Negatively-acting growth regulatory (NGR) elements are those that, when expressed in or provided to a cell, lead to suppression of cell growth. Positively-acting growth regulatory (PGR) elements are generally those products, or genes and their products which, when expressed in or provided to a cell, stimulate its proliferation.

### Negative Growth Regulatory (NGR) Elements

The expression of negatively-acting growth genes is important in regulating and controlling cell proliferation. Examples of NGR elements are several genes which have been identified, the expression of which is induced in cells that are quiescent or in the resting state. These genes may be termed negative growth regulatory (NGR) genes. Down regulation of these negatively-acting growth regulatory genes results in the removal of the inhibition of cell proliferation. In addition to biological or physiological phenomena which result in the inactivation of these genes or the gene products, certain pathological conditions have been identified in which the negatively-acting growth regulatory genes are inactive, or absent. These genes may be inactive due to deletions, mutations or downregulation of the gene, or due to the overabundance of a factor which binds or inactivates the gene product and, therefore, prevents, partially or completely, the expression of the gene or the function of its product, and thus, its ability to block or inhibit cell proliferation.

The human retinoblastoma gene, Rb1, is the prototype of this class of tumor suppressor genes, herein termed Negative Growth Regulatory (NGR) genes in which the absence of both alleles of the gene in a cell or the inhibition of the expression of the gene or its gene product will lead to neoplastic or abnormal cellular proliferation. It has been demonstrated at the molecular level that the loss or inactivation of both alleles of the RB1 gene is involved in the clinical manifestation of tumors such as retinoblastoma and clinically related tumors, such as osteosarcomas, fibrosarcoma, soft tissue sarcoma and melanoma.

Additionally, loss of the function of the Rb-1 gene has also been associated with other types of primary cancer such as primary small cell lung carcinoma (SCLC), soft tissue sarcomas, breast carcinoma, cervical carcinoma and prostate carcinoma.

Introduction of the Rb-1 gene into a tumor cell that has lost the Rb-1 gene, resulted in the suppression Of the growth, of the tumor. For instance, when the cDNA of Rb-1 was delivered into the osteosarcoma cell line SAOS2 and the prostate cancer cell line DU 145, tumor cells that have lost their endogenous Rb gene, the growth of some of these tumor cells was specifically suppressed in vivo (Huang et al., (1988) Science 242:1563; Bookstein et al., (1990) Science 247:712). These authors concluded that, while the retinoblastoma gene specifically suppressed the tumorigenic phenotype, it had no effect on normal cells.

### Positively-Acting Growth Regulator (PGR) Elements

Positively-acting growth regulatory (PGR) elements include all the proto-oncogenes, the expression of which has been shown to be activated in some forms of cancer and during cell proliferation in normal cells and in certain pathological cell proliferative diseases. Activation of the expression of proto-oncogenes is indispensable for cell proliferation. Many of the specific genes in this class have been found to code for growth factors or their receptors. The expression of some proto-oncogenes has been found to be tightly linked to the proliferative state when a cell is exposed to growth factors. Many growth factors, such as platelet derived growth factor (PDGF), epidermal growth factor (EGF), insulin, and transferrin, to name a few, are required for cell proliferation. Cells which do not express the specific receptor molecule for a growth factor cannot proliferate even when the growth factor is presented to the cell. On the other hand, cells in which the normal negative regulation of the expression of the receptor is interfered with, resulting in uncontrolled expression of the receptor, will proliferate in an uncontrolled manner. When quiescent cells are exposed to platelet derived growth factor (PDGF) or epidermal growth factor (EGF), the synthesis of new mRNA of several proto-oncogenes including c-fos and c-myc is induced. Similarly, resting hepatic cells can be stimulated to grow in vivo by partial hepatomy. This stimulation of growth is associated with an elevated expression of several proto-oncogenes including c-myc. Activation of these PGR genes is required for normal, as well as abnormal or pathological, cell proliferation.

Pathological cell proliferation disorders or diseases are often associated with inappropriate activation of proto-oncogenes. The level of c-myc expression, for example, is highly amplified in the non-lymphocytic leukemia cell line HL-60. When HL-60 cells are induced to stop proliferation by chemical inducers, such as retinoic acids and dimethyl sulfoxide, the level of c-myc is downregulated. On the other hand, exposure of HL-60 cells to a DNA construct which is complementary to the 5' end of c-myc or c-myb causes arrest of translation of these mRNAs and c-myc or c-myb protein expression is down regulated, resulting in the arrest of cell proliferation and differentiation of the treated cells (Wickstorm et al (1988) Proc. Natl. Acad. Sci 85:1028; Anfossi et al (1989) Proc. Natl. Acad. Sci 86:3379).

### Summary of the Invention

In one aspect, the present invention provides a generalised approach to the treatment of inappropriate or pathological cell growth in benign proliferative diseases.

Accordingly, the present invention provides the use of DNA encoding a retinoblastoma gene which directs the expression of an encoded peptide, in the preparation of a medicament for use in a method of treatment of pathological benign cellular proliferative diseases by administration of said medicament to a cell.

Preferably, the DNA encoding the retinoblastoma gene is contained within a recombinant expression vector.

The expression vector is preferably a viral vector, more preferably a retroviral vector. In a most preferred embodiment, the retroviral vector is defective and will not transform non-proliferating cells. The expression vector may be a plasmid, preferably encapsulated in a liposome.

Preferably, the medicament is for use in the treatment of abnormally proliferating cells associated with a disease selected from the group consisting of benign proliferative skin diseases, psoriasis, ichthyosis, papillomas, basal cell carcinomas, squamous cell carcinoma, fibroproliferative diseases, vasoproliferative diseases, dermatoproliferative diseases and neoplastic diseases of the eye.

Preferably, the proliferating cells contain at least one active allele of a negative growth regulatory gene but still proliferate inappropriately.

Other and further aspects, features, and advantages of the present invention will be apparent from the following description of the presently preferred embodiments of the invention given for the purpose of disclosure.

### Brief Description of the Figures

Figure 1 shows the construction and the structure of plasmid HuBAcpr-1-neo-Rb containing part or all of the Rb-1 cDNA under the control of a human β actin promoter.
Figure 2 demonstrates the effect of transfected plasmids on proliferation of the human fibroblast cell line WS1.
Figure 3 shows the schematic representation of the retroviral vector containing the Rb-1 gene.

### Detailed Description of the Preferred Embodiments

### Definitions

The present invention uses a DNA construct of the Rb-1 gene, preferably incorporated into a vehicle or vector preferably under the promotion of a strong promoter. Preferably the DNA construct of the present invention is delivered to the target cells utilising the MuLV based retroviral vector.

The term "functional expression of the gene" is meant to include the suppression of transcription of the gene, the degradation of the gene transcript (pre-message RNA), the inhibition of splicing, the destruction of the message RNA, the prevention of translation of the message RNA, the prevention of the post-translational modifications of the protein, the destruction of the protein, or the inhibition of the normal function of the protein.

The term "transfected plasmid" is meant to include the bacterial plasmid which contains the DNA encoding a retinoblastoma gene, to be carried (transfected) into the cell of choice.

The term "gene therapy" is meant to include the insertion of part or all of the DNA into a cell, group of cells, tissue, pathologic lesion, organ or organism for the purpose of modulating gene expression, and/or function of the gene product.

The term "prophylactic gene therapy" is meant to include genes which may be used for partial or total inhibition or prevention of disease and the spread of disease and also is meant to include genes which may be used to supplement or replace absent or defective negative growth in cell, tissues or germlines.

The term "cell proliferative disease" is meant to include any human or animal disease or disorder, affecting any one or any combination of organs, cavities or body parts, which is characterized by single or multiple local abnormal proliferations of cells, groups of cells or tissue(s), which are benign.

The term "prokaryote" is meant to include all bacteria which can be transformed with the DNA for the expression of the recombinant molecules of the present invention.

The term "eukaryote" is meant to include all yeasts, fungi, animal and plant cells which can be transformed with the DNA for the expression of the recombinant molecules of the present invention.

The DNA for DNA constructs of the present invention can be synthetic or may be derived from any mammalian species. All that is required is that the genetic sequence for the retinoblastoma gene be functionally expressed in the prokaryotic or eukaryotic organism. Preferred is synthetic DNA.

A recombinant DNA molecule coding for the DNA constructs of the present invention can be used to transform a host using any of the techniques commonly known to those of ordinary skill in the art.

Methods for preparing fused, operably linked genes and expressing them in bacteria are known and are shown, for example, in U.S. Patent No. 4,366,246, herein incorporated by reference. The genetic constructs and methods described therein can be utilized for construction of the DNA constructs of the present invention and transfection in prokaryotic or eukaryotic hosts.

Prokaryotic hosts may include Gram negative as well as Gram positive bacteria, such as E. coli, S. tymphimurium, Serratia marcescens, and Bacillus subtilis.

Eukaryotic hosts may include yeasts such as Pichia pastoris or mammalian cells.

In general, expression vectors containing promoter sequences which facilitate the efficient transcription of the inserted DNA fragment are used in connection with the host. The expression vector typically contains an origin of replication, promoter(s), terminator(s), as well as specific genes which are capable of providing phenotypic selection in transformed cells. The transformed hosts can be fermented and cultured according to means known in the art to achieve optimal cell growth.

Examples of promoters which can be used in the invention include, but are not limited to: human β actin promotor, metallothionin promotor, SV40 origin of replication, and MMTV LTR promotor and MuLV LTR promotor. Examples of some of the plasmids or bacteriophage which can be used in the invention are listed in Maniatis et al., Molecular Cloning, Cold Spring Harbor Laboratories, 1982, and others are known to those of skill in the art and can be easily ascertained.

A gene is a DNA sequence which encodes through its template or messenger RNA a sequence of amino acids characteristic of a specific peptide. The term cDNA includes genes from which the intervening sequences have been removed. The term "recombinant DNA" (rDNA) is meant to include a molecule that has been recombined by splicing cDNA or genomic DNA sequences in vitro.

A cloning vehicle is a plasmid or phage DNA or other DNA sequence which is able to replicate in a host cell which is characterized by one or a small number of endonuclease recognition sites at which such DNA sequences may be cut in a determinable fashion without loss of an essential biological function of the DNA, and which contains a marker suitable for use in the identification of transformed cells. Markers, for example, are tetracycline resistance or ampicillin resistance. The word "vector" is sometimes used for a cloning vehicle.

An expression vehicle is a vehicle similar to a cloning vehicle but which is capable of expressing a given structural gene in a host, normally under control of certain control sequences.

The term "individual" is meant to include animals and humans.

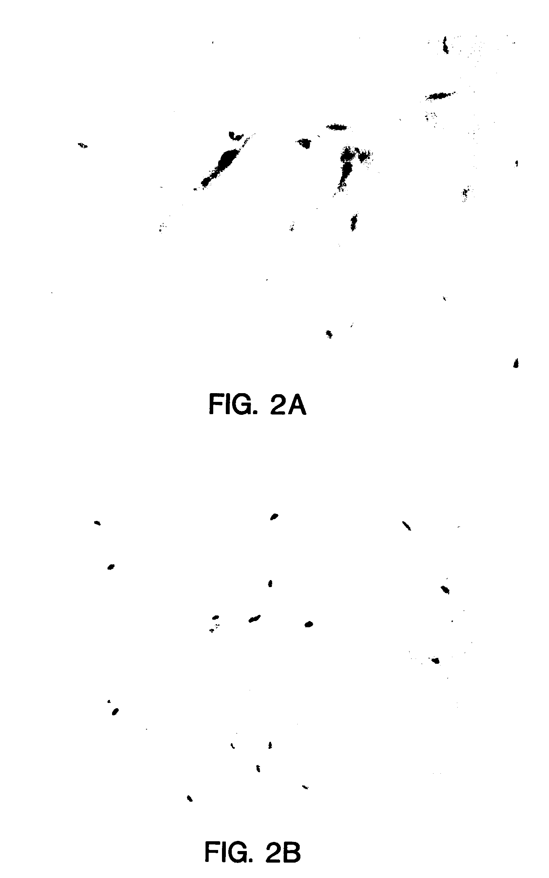

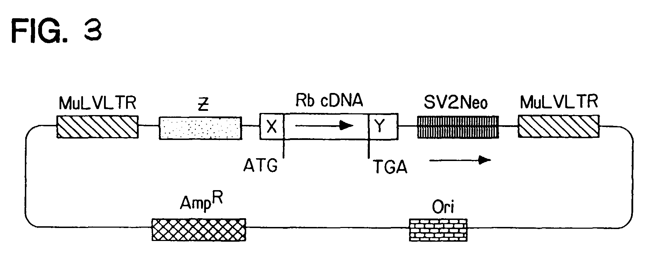

The term "biologically inhibiting" or "inhibition" of the growth of proliferating cells is meant to include partial or total growth inhibition and also is meant to include decreases in the rate of proliferation or growth of the cells. The biologically inhibitory dose of the DNA of the present invention may be determined by assessing the effects of the DNA on target malignant or abnormally proliferating cell growth in tissue culture (see Examples 3-4 and Figures 2-4), tumor growth in animals and cell culture or any other method known to those of ordinary skill in the art.

Administration of the DNA used in the invention may be by topical, intraocular, parenteral, oral, intranasal, intravenous, intramuscular, subcutaneous, or any other suitable means. The preferred method of administration for treatment of ocular diseases is intraocular or periocular injection. The preferred method of administration for treatment of skin cell proliferative diseases is by topical application or subcutaneous injection.

In another embodiment, the DNA constructs of the present invention may be delivered to the focal proliferative disease directly. In the case of ocular proliferative disease, the DNA constructs of the present invention may be directly injected into the eye. The DNA constructs of the present invention may be delivered directly to focal disease sites in internal organs, body cavities and the like by use of imaging devices used to guide the injecting needle directly to the disease site. The DNA constructs of the present invention may also be administered to disease sites at the time of surgical intervention.

The DNA dosage administered is dependent upon the age, clinical stage and extent of the disease or genetic predisposition of the individual, location, weight, kind of concurrent treatment, if any, and nature of the pathological or malignant condition. The effective delivery system useful in the method of the present invention may be employed in such forms as capsules, tablets, liquid solutions, suspensions, or elixirs, for oral administration, or sterile liquid forms such as solutions, suspensions or emulsions. Any inert carrier is preferably used, such as saline, or phosphate-buffered saline, or any such carrier in which the compounds used in the method of the present invention have suitable solubility properties.

Preferably, for intraocular delivery and for delivery to other localized disease sites, delivery systems useful in the method of the present invention may be employed in such sterile liquid forms such as solutions, suspensions or emulsions. For topical use it may be employed in such forms as ointments, creams or sprays. Any inert carrier is preferably used, such as saline, or phosphate-buffered saline, or any such carrier in which the compounds used in the method of the present invention have suitable solubility properties.

For local administration to cells, the DNA of the present invention may be administrated by any method known to those of skill in the art including, but not limited to, transfection, electroporation, microinjection of cells, or in vehicles such as liposomes, lipofectin or as naked DNA or RNA. The DNA of the present invention may be delivered by known gene delivery systems such as, but not limited to, retroviral vectors (Gilboa (1982) J. Virology 44:845; Hocke (1986) Nature 320:275; Wilson et al., Proc Natl Acad Sci USA 85:3014), vaccinia virus system (Chakrabarty et al., (1985) Mol. Cell Biol. 5:3403) or other efficient DNA delivery systems (Yates et al., (1985) Nature 313:812) known to those of skill in the art. These references are exemplary only and are incorporated herein by reference. In order to specifically deliver or transfect cells which are abnormally proliferating and spare non-dividing cells, it is preferable to utilize a retrovirus delivery system known to those of skill in the art. Since host DNA replication is required for retroviral DNA to integrate and the retrovirus will be unable to self replicate due to lack of the retrovirus genes needed for its life cycle, utilizing such a retroviral delivery system for the DNA of the present invention will target said DNA to abnormally proliferating cells and will spare non-dividing normal cells from any intervention.

The DNA of the present invention may be administered in any biologically effective carrier. The biologically effective carriers may include retroviruses, liposomes, and any other transfection mechanism capable of introducing foreign DNA into the genome of the cell. Such transfection mechanisms are known to those of skill in the art. The carrier may also include any agent or solvent with which the constructs of the present invention are compatible and which are non-toxic to the individuals or cells treated at the amounts administered.

There is a wide variety of pathological cell proliferative conditions for which the method of the present invention will provide therapeutic benefits. These pathological conditions may occur in almost all cell types capable of abnormal cell proliferation. Among the cell types which exhibit pathological or abnormal growth are (1) fibroblasts, in which case the diseases are known as fibroproliferative disorders; (2) vascular endothelial cells, in which case the diseases are known as vasoproliferative disorders and (3) epithelial cells in which case the diseases are known as dermatoproliferative diseases. It can be seen from the above that the method of the present invention is useful in treating local or disseminated pathological conditions in all or almost all organ and tissue systems of the individual.

For instance, in the eye alone, the method of the present invention may be utilized to treat such a wide variety of pathologic disease states which are due to abnormal proliferation of normal or benign cells or tissues including, but not limited to, the following fibroproliferative, vasoproliferative and/or neoplastic diseases of the eye: retinopathy of prematurity, proliferative vitreoretinopathy, proliferative diabetic retinopathy, capillary hemangioma, choroidal neovascular nets, subretinal neovascular nets, senile macular degeneration due to subretinal, neovascularization, corneal neovascularization, macular pucker due to epiretinal membrane proliferation, adult cataracts due to nuclear sclerosis, fibrous ingrowth following trauma or surgery, optic nerve gliomas, angiomatosis retinae, neovascular glaucoma, cavernous hemangioma, rubeosis iridis, sickle cell proliferative retinopathy, epithelial downgrowth after eye surgery or injury, after-cataract membrane, papilloma, retinal neovascularization in thalassemia, subretinal neovascularization due to pseudoxanthoma elasticum, and neurofibromatosis type l and 11 and pseudotumor of the orbit.

Other benign cell proliferative diseases for which the present invention is useful include, but are not limited to, psoriasis, ichthyosis, papillomas, basal cell carcinomas, squamous cell carcinoma, and Stevens-Johnson Syndrome.

According to the method of the present invention, one can manipulate the cellular proliferative process by the introduction of the DNA to prevent or inhibit abnormal proliferation in a wide variety of cell proliferative diseases. The manipulation of the proliferative process may be accomplished by introducing into a target proliferating cell a DNA construct which encodes a retinoblastoma gene.

To illustrate the use of the DNA to inhibit abnormal cell proliferation in non-malignant or non-cancer cells by the method of the present invention, the human retinoblastoma gene, Rb-1, was introduced into a normal human fibroblast cell line. As described more fully in Example 2, introduction of a human Rb cDNA into normal human fibroblast, WS1, led to the cessation of cell growth. Similarly, introduction of this human retinoblastoma gene into several different tumor cell types led to the suppression of growth of these cells in vitro or in vivo as further described in Examples 3 and 4.

We and others have shown that the human retinoblastoma gene product is heavily phosphorylated when cells are proliferating at the G1/S boundary and the S phase of the cell cycle (Buckovich et al., (1989) Cell 58:1097; Mihara et al., (1989) Science 246:1300; Decaprio (1989) Cell 58:1085 and Chen et al., (1989) Cell 58: 1193), but is unphosphorylated or underphosphorylated when cells are not proliferating at the G1 phase. Exposure of a resting cell to mitogens results in the activation of a kinase complex which phosphorylates the Rb protein. As a result, the cell enters into DNA replication and mitosis. Thus, in order for a cell to initiate DNA replication, inactivation of the human retinoblastoma gene product by phosphorylation may be necessary.

In one embodiment of the present invention, the normal cellular phosphorylation of the Rb-1 protein can be inhibited or interfered with by either introduction of additional copies of the RB-1 gene, causing over production of the Rb-1 protein, or by mutagenizing the Rb-1 gene so that it cannot be phosphorylated.

When a highly expressed retinoblastoma gene was introduced into a normal non-tumor cell, abundant amounts of the retinoblastoma protein were expressed. The treated cell therefore contains an excessive amount of active Rb protein and proliferation is arrested. The retinoblastoma protein which normally exists in a multiply phosphorylated form becomes dephosphorylated when proliferating cells were growth arrested by serum depletion. This excessive amount of Rb protein may overwhelm the capacity of the cellular kinase to phosphorylate and inactivate the Rb gene product. Thus, in one embodiment, cell proliferation may be arrested or inhibited by the introduction of the Rb-1 gene into a cell. In order to insure the growth arrest process, the genes to be introduced should be placed under the control of a strong promoter. The human β-actin promoter is one such strong promotor. The construction of a DNA construct comprising the Rb gene under the control of a strong promotor such as the human β actin gene is shown in Example 1. However, it will be obvious to one of skill in the art that other strong promotors may also be utilized in practicing the present invention.

In another embodiment, the phosphorylation site the Rb-1 gene is mutagenized before introduction into a cell. There are 7-12 potential phosphorylation sites on the Rb protein. Mutation of the serine or threonine coding sequences in the Rb-1 cDNA into alanine or valine or others would therefore lead to the production of a permanently active Rb protein which cannot be inactivated by phosphorylation. Thus, the host cell will not be able to inactivate the Rb protein by phosphorylation. Introduction of such a mutated Rb-1 gene into a cell will therefore lead to growth arrest.

One can administer more than one of the DNA constructs of the present invention simultaneously to the same site.

Having now generally described the invention, a more complete understanding can be obtained by reference to the following specific examples. These examples are provided for purposes of illustration only and are not intended to be limiting unless otherwise specified.

### Example 1

### Construction of the Rb-1 cDNA Expression Plasmid

The general procedure for plasmid construction and DNA preparation was as described by Maniatis et al. (Maniatis, T., Fuitsch, E.F., and Senbrook, J. (1982) Molecular Cloning: A Laboratory Manual (Cold Spring Harbor Laboratory, Cold Spring Harbor, N.Y., U.S.A.). All plasmids were grown in the Escherichia coli host DH5-α purchased from Bethesda Research Laboratory (BRL). The structure and properties of the human β-actin expression vector system, pHBAPr-1-neo has been described (Gunning et. al., (1987) Proc. Natl. Acad. Sci. USA 84, 4831-4835). The Rb-1 cDNA sequence has been described previously (Fung et al., (1989) in Oncogenes, chapter 13, Molecular Biology of the Human Retinoblastoma Gene, Benz, and Lin, (eds.) Kluwen Academic Publishers.)

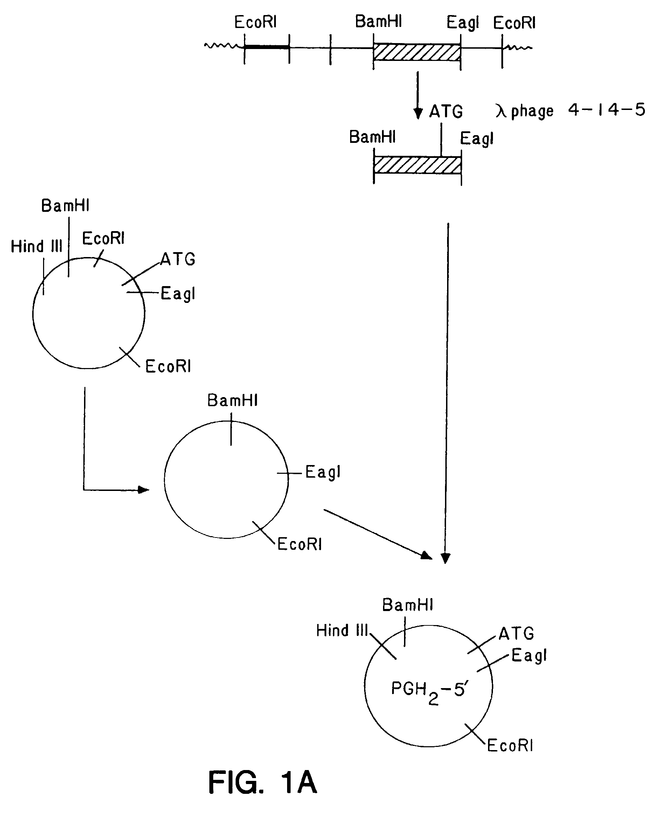

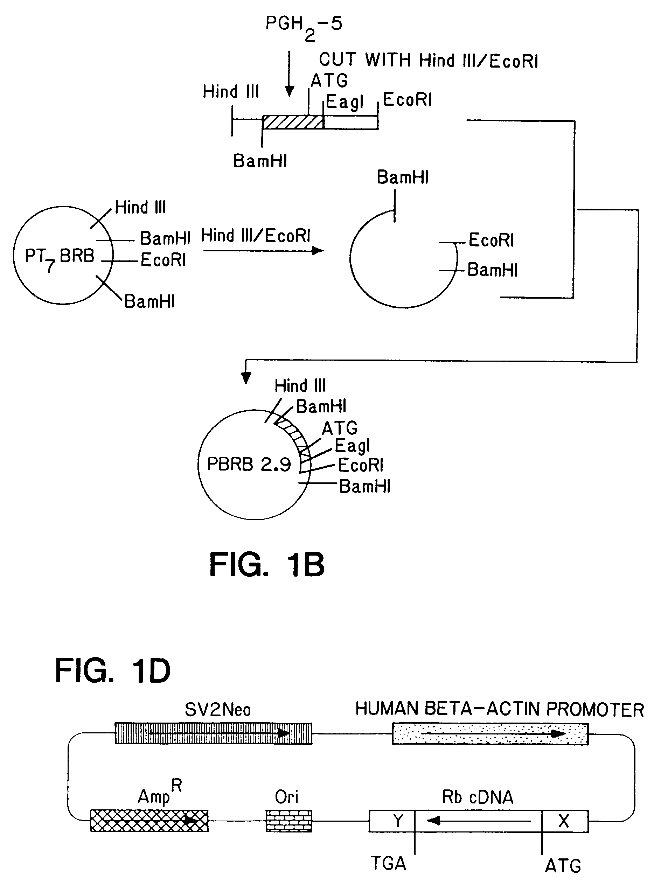

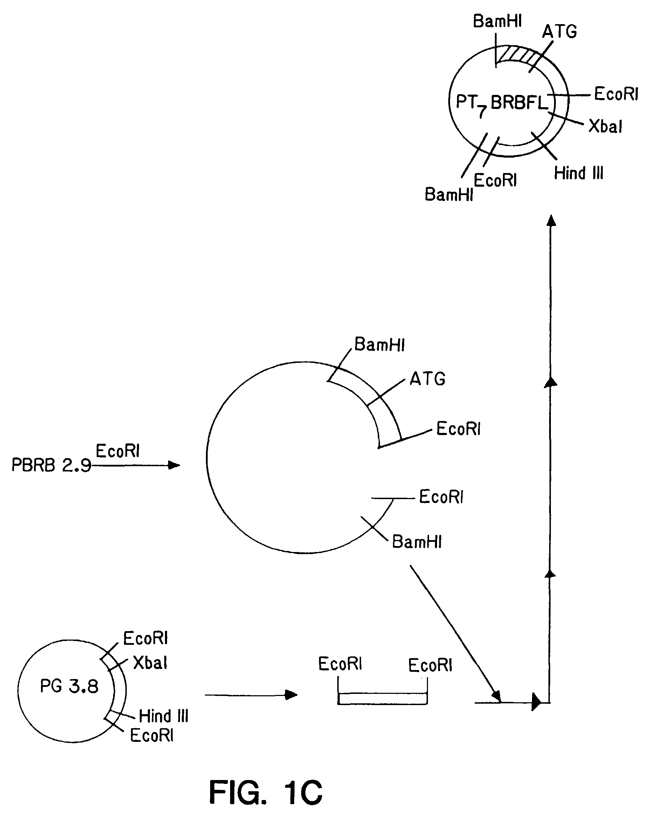

The construction of the HuBAcpr-1-neo-Rb-8342 is schematically demonstrated in Figure 1, Panels A-C. For the construction of HuBAcpr-1-neo-Rb-8342, a BamH1-Eag1 DNA fragment containing part of the first exon was excised from the λ phage clone 4-14-5 (T'Ang et al (1989) Oncogene 4, 401-407) and ligated to the 5' end of Rb-1 cDNA plasmid PGH2 (Fung et al, (1987) Science 236:1657-1661) at the BamH1-Eag1 sites. (See Figure 1, Panel A) The resulting plasmid was grown up and the Hind III EcoR1 fragment containing the Rb-1 promotor region and the 5' portion of the Rb-1 cDNA was transferred to a plasmid PT7BRB at the Hind III and EcoR1 site (See Figure 1, Panel B), generating the plasmid pBRB2.9. The 3.8 kb 3' half of the Rb cDNA was excised with EcoR1 from PG 3.8 (Fung et al (1987) Science 236:1657-1661) and subcloned into the EcoR1 site of pBRB2.9 to generate PT7BRBFL. (See Figure 1, Panel C) Finally, the BamH1 fragment containing the Rb-1 cDNA and its promotor was subcloned into the HBApr-1-neo at the BamH1 site to generate HuBAcpr-1-neo-Rb-8342.

For the construction of HuBAcpr-1-neo-RB-PQ, a double stranded synthetic linker (in which only one strand is shown here):
5' ACGGATC CGCGTC ATG CCG CCC AAA ACC CCC CGA AAA ACG GCCG 3'
was digested with BamH1 and Eag1 and subcloned into the BamH1/Eag1 site of the plasmid PT7BRBFL (See Figure 1, Panel C), replacing the Rb promotor. This plasmid was then partially digested with the enzyme ppum1 to linearize at nucleotide # 2797-2799. A synthetic linker containing a poly A signal (AATAAA) and a BamH1 site flanked by ppum1 sites was inserted into the ppum1 site of the linearized PT7BRBFL. The resulting plasmid was grown up, and the BamH1 fragment containing the Rb1 cDNA coding region was excised and then inserted into the BamH1 site of the plasmid HBAcpr-1-neo to generate the plasmid HuBAcpr-1-neo-Rb-PQ.

Figure 1D shows the general structure of plasmid HuBAcpr-1-neo-Rb containing part or all of the Rb-1 cDNA under the control of a human β-actin promoter.

Two different HuBAcpr-1-neo-Rb expression plasmids were constructed. For HuBAcpr-1-neo-Rb-8342, X is the BamH1 2kb fragment in front of the first ATG of the longest open reading frame (T'Ang et al., (1989) Oncogene 4:401) and Y is the entire 3' untranslated region of Rb cDNA.

For HuBAcpr-1-neo-Rb-PQ, X is a synthetic linker (5-GATCCGCGTC-3) placed in front of the first ATG and Y is a synthetic linker containing a poly A tail signal AATAAA, behind nucleotide #2801.

### Example 2

### Effect of Transfected Plasmids on Proliferation of the Human Fibroblast Cell Line WS1

When normal keratinocytes are exposed to the tumor growth factor β (TGFβ) which inhibits the growth of many cell types, the retinoblastoma protein in the keratinocyte rapidly becomes dephosphorylated and the keratinocyte ceases to grow. The SV 40 large T antigen, which presumably inactivates the retinoblastoma gene product by binding to it, can only bind to the under-phosphorylated (active) form of the retinoblastoma protein. Therefore, as a cell is induced into the growth arrest state, an associated change is the dephosphorylation of the retinoblastoma protein. Restimulation of arrested cells to grow by exposure to mitogens results in heavy phosphorylation of the retinoblastoma protein prior to the initiation of DNA synthesis. The fact that this is observed in normal and tumor cells suggests that the inactivation of the retinoblastoma protein by phosphorylation is required for cell proliferation to occur. The retinoblastoma protein may therefore be involved in the growth retardation or arrest of normal and tumor cells.

To study effect of the RB cDNA on cell growth, HuBAc-1-neo-Rb-PQ was transfected into the normal human fibroblast cell line WS1. As a control, a DNA fragment containing the neomycin resistant gene under the control of the RSV LTR was excised from the plasmid PATV-6D3A, synthetic sal 1 linkers were ligated to the fragment, digested with sal 1 and then ligated to PPVU0 (Kalderon and Smith, (1984) Virology 139:109-137) to generate PPVUO-Neo. This plasmid, PPVUO-Neo, which contains the gene for the SV4O large T antigen was then transfected in order to monitor the efficiency of transfection. For transfection, 100ug of each of the plasmid DNA were mixed with 10⁷ exponentially grown WS1 cells in a final volume of 0.8ml of RPM1 1640 medium (Gibco) plus 10% FCS in an electroporation chamber unit Cell-Porator™ (BRL). Electroporation was done at 200 volt and 1180uF. The electroporated cells were allowed to recover at room temperature for 10 minutes and were then diluted in RPM1 1640 plus 10% fetal calf serum (FCS) and plated out in 60 mm dishes at a cell density of 2 X 10⁴ cells/cm². The cells were allowed to attach and grow in the same medium for two days. Thereafter, the medium was changed to RPM1 1640 + 10% FCS + 15ug/ml G418(GIBCO). Every three days, duplicate dishes were taken for histoimmunochemical staining using either a rabbit polyclonal anti-Rb protein antibodies RB1-ABA1 or RB1-AB18 (Mihara K. et al (1989) Science 246:1300) (Figure 2A), or with the mouse monoclonal anti-SV40 large T antigen antibody PAB 101 (Figure 2B). The staining procedure described in Example 8 was utilized. As can be seen from Figure 2A, 13 days after transfection and selection in G418 medium, the WS1 cells that were expressing the transfected Rb cDNA plasmid HuBAcpr-1-neo-Rb-PQ stained intensely with the anti-Rb protein antibodies. However, the cells that expressed the Rb protein remained as single cells and did not divide. In contrast, cells that expressed the transfected SV40 large T antigen (SVLT) continued to divide into colonies as shown by the group of cells stained positive with the mouse anti SVLT antibody in Figure 2B.

Thus, over expression of the Rb cDNA in a cell led to extreme retardation or arrest of cell growth.

### Example 3

### Immunohistochemical Localization of the Rb Protein in Cells

Cells were cultured in RPM1 1640 medium supplemented with 10% FCS and 0.1% Penicillin/streptomycin. The cells were rinsed twice with cold PBS (phosphate buffered saline). The cells were fixed with a solution containing 5% acetic acid and 95% ethanol at 4°C for 12 hours.

After fixing, the cells were washed twice in cold PBS. The nonspecific binding was blocked by incubating the cells in a PBS solution containing 1% normal horse serum, 3% bovine serum albumin, and 0.2% Triton X-100 for 1 hour at 4°C. The cells were then rinsed twice with PBS. The cells were then incubated with either a 4°C solution containing a 1:1600 dilution of RB1-AB18 or a 1:100 dilution of RB1-ABA1 in PBS for 1 hour at 37°C. The cells were then washed sequentially with cold PBS and PBS containing 1% Triton X-100 and PBS. The specific binding of the RB1 antibodies to the cells was visualized by incubation with biotinylated goat anti-rabbit IgG antibodies (diluted 1:200 in PBS) for 1 hour at 37°C. After washing the cells three times with cold PBS, the cells were incubated with ABC reagent (avidin: biotin complex) at 37°C for 30 minutes, washed with cold PBS and then incubated with freshly prepared DAB (3,3'-Diamixobenzidine - tetrahydrochloride) solution (6mg of DAB in 10 ml of 0.05 M TRIS-HCl, pH 7.6, 0.3% sodium azide and 10ul hydrogen peroxide) at room temperature for 4 minutes. The cells were then washed with distilled water and examined under a light microscope.

## Claims

1. Use of DNA encoding a retinoblastoma gene which directs the expression of an encoded peptide, in the preparation of a medicament for use in a method of treatment of pathological benign cellular proliferative diseases by administration of said medicament to a cell.

2. Use according to claim 1, wherein said DNA encoding the retinoblastoma gene is contained within a recombinant expression vector.

3. Use according to claim 2, wherein said expression vector is a viral vector.

4. Use according to claim 2, wherein said expression vector is a retroviral vector.

5. Use according to claim 2, wherein said expression vector is a plasmid.

6. Use according to claim 5, wherein said plasmid is encapsulated in a liposome.

7. Use according to any one of claims 1 to 6, wherein the treatment is of abnormally proliferating cells associated with a disease selected from the group consisting of psoriasis, benign proliferative skin diseases, ichthyosis, papilloma, basal cell carcinoma, squamous cell carcinoma, fibroproliferative diseases, vasoproliferative diseases, dermatoproliferative diseases, and neoplastic diseases of the eye.

8. Use according to any one of claims 1 to 7, wherein said proliferating cells contain at least one active allele of a negative growth regulatory gene but still proliferate inappropriately.

## Patentansprüche

1. Verwendung einer für ein Retinoblastomgen codierenden, die Expression eines codierten Peptids richtenden DNA bei der Herstellung eines Arzneimittels zur Verwendung bei einem Verfahren zur Behandlung pathologischer, gutartiger, Zellproliferativer Erkrankungen durch Verabreichung des Arzneimittels an eine Zelle.

2. Verwendung nach Anspruch 1, wobei die für das Retinoblastomgen codierende DNA in einem rekombinanten Expressionsvektor enthalten ist.

3. Verwendung nach Anspruch 2, wobei der Expressionsvektor ein viraler Vektor ist.

4. Verwendung nach Anspruch 2, wobei der Expressionsvektor ein retroviraler Vektor ist.

5. Verwendung nach Anspruch 2, wobei der Expressionsvektor ein Plasmid ist.

6. Verwendung nach Anspruch 5, wobei das Plasmid in ein Liposom eingekapselt ist.

7. Verwendung nach einem der Ansprüche 1 bis 6, wobei die Behandlung abnorm proliferierende Zellen, die mit einer Erkrankung, ausgewählt aus der Gruppe, bestehend aus Psoriasis, gutartigen proliferativen Hauterkrankungen, Ichthyosis, Papillom, Basalzellencarcinom, squamösem Zellcarcinom, fibroproliferativen Erkrankungen, vasoproliferativen Erkrankungen, dermatoproliferativen Erkrankungen und neoplastischen Erkrankungen des Auges, verbunden sind, betrifft.

8. Verwendung nach einem der Ansprüche 1 bis 7, wobei die proliferierenden Zellen mindestens ein aktives Allel eines negatives Wachstum regulierenden Gens enthalten, aber noch unangemessen proliferieren.

## Revendications

1. Utilisation d'un ADN codant un gène de rétinoblastome qui dirige l'expression d'un peptide codé, dans la préparation d'un médicament destiné à un procédé de traitement de maladies cellulaires prolifères bénignes par administration dudit médicament dans une cellule.

2. Utilisation selon la revendication 1, dans laquelle l'ADN codant le gène du rétinoblastome est contenu à l'intérieur d'un vecteur d'expression recombinée.

3. Utilisation selon la revendication 2, dans laquelle ledit vecteur d'expression est un vecteur viral.

4. Utilisation selon la revendication 2, dans laquelle le vecteur d'expression est un vecteur rétro-viral.

5. Utilisation selon la revendication 2, dans laquelle le vecteur d'expression est un plasmide.

6. Utilisation selon la revendication 5, dans laquelle le plasmide est encapsulé dans un liposome.

7. Utilisation selon l'une quelconque des revendications 1 à 6, dans laquelle le traitement concerne des cellules à prolifération anormale liée à une infection sélectionnée dans le groupe constitué par le psoriasis, des affections dermatologiques prolifères bénignes, l'ichthyose, le papillome, le carcinome cellulaire basal, le carcinome cellulaire squameux, les maladies fibroprolifères, les maladies vasoprolifères, les maladies dermatoprolifères et les affections néoplastiques de l'oeil.

8. Utilisation selon l'une quelconque des revendications 1 à 7, dans laquelle lesdites cellules prolifères contiennent au moins un allèle actif d'un gène régulateur de croissance et continuent néanmoins à proliférer de façon inappropriée.
